# EUROPEAN PATENT APPLICATION

(11) **EP 1 348 962 A1**
(43) Date of publication of application: **01.10.2003**
(21) Application number: 03251947.2
(22) Date of filing: 27.03.2003
(51) Int. Cl.: G01N 33/543

(54) **Biomolecular sensors and detection methods**

(30) Priority: 28.03.2002 US 108672
(71) Applicant: Agilent Technologies, Inc., Palo Alto, CA 94306 (US)
(72) Inventor: Roitman, Daniel B., Menlo Park, CA 94025 (US); Tom-Moy, May, San Carlos, CA 94070 (US); Inaoka, Seiji, San Jose, CA 95125 (US); Ach, Robert A., San Francisco, CA 94131 (US)
(74) Representative: Tollett, Ian

(57) **Abstract**

The invention provides methods and sensors for detecting target biological molecules. Biosensors feature photoactivatable charge separation moieties capable of generating electron-hole pairs upon photoinduction. Photoinduced charge carriers participate in redox reactions that are detectable, for example, by optical, chemical, or electronic means.

## Description

The present invention relates to sensors and methods for detecting target biological molecules bound to probe molecules on a substrate. In particular, biosensors of the invention feature photoactivatable charge separation as a source of electron-hole pairs. The charge separation is preferably localized to bound target and probe molecules of interest where the charge carriers take part in detectable redox reactions.

Recent developments in biotechnology have enabled massively parallel screening methods by which, for example, test samples can be assayed for a large number of components and multiple test samples can be assayed simultaneously for one or more components.

Development of microarrays has revolutionized gene expression analysis in research and diagnostic applications, allowing the monitoring of a large number of genes simultaneously. In a microarray, such as a gene chip, thousands of different DNA fragments or probes can be immobilized on a defined surface. The unique sequence of the immobilized fragment at each coordinate is known, so that when a target sample is introduced to the array and hybridization is found to occur, the identity of gene fragments within the target sample can be deduced. This approach is known as spatial multiplexing because the target molecules are identified based on the spatially defined positions of the probe molecules. Alternatively, identical probe molecules may be immobilized at multiple locations of an array and multiple samples screened in parallel. Here, positional information is associated with the sample rather than with the immobilized screening reagent.

Microarray technology is also applicable to analysis of proteins, protein-protein interactions and enzyme catalysis. Protein function has become a major target of biological research as genomic analysis has resulted in the accumulation of vast amounts of protein sequence information, but little data beyond sequence homology to guide identification of function. Accordingly the capacity to analyze the physical and biochemical properties of large numbers of proteins is becoming increasingly important. Thus, systems that allow massively parallel analyses of protein and protein interactions are of great value.

The functions of biological molecules in physiological pathways is also of great interest. Microfluidic devices in which interconnected fluid pathways and reaction chambers are engineered to provide a "lab on a chip," have been used to carry out proteomic analysis. These microfluidic devices can be integrated with arrays.

Fluorescence labels (e.g., fluorescein, rhodamine, phycorerythrin) linked to a target or an intercalation dye which selectively binds to a double stranded DNA hybrid are typically used to detect hybridization of a DNA or RNA target on a DNA array. Fluorescent probes are also used to identify proteins captured on protein chips. Spectral multiplexing, by making use of fluorescence labels differentiable by color, can be used to simultaneously detect a small number of different targets. However, these methods require the use of fluorescence scanners and other specialized equipment and reagents. Moreover, the applicability of spectral multiplexing has been limited by the small number of molecules that could be distinguished during detection, due to the broad emission wavelength of conventional labels and their short-lived fluorescence.

Immobilized gold nanoparticles have been used to catalyze reduction and deposition of additional gold or silver, but the technique is not compatible with color multiplexing or suitable for photo-electrochemical detection.

Accordingly, there remains a need for versatile and inexpensive methods for analysis of large numbers of samples that can take advantage of inexpensive and versatile scanning and analysis equipment.

The invention provides a sensor for detecting a target biomolecule bound to a probe biomolecule on a substrate. The sensor comprises a photoinducible charge-separation moiety that effects charge separation upon photoinduction, an electron donor, and an electron acceptor. The electron acceptor is capable of providing detectable photoreduction indicating the presence of the bound target molecule upon excitation of the charge-separation moiety.

The invention further provides a method for detecting a target biomolecule bound to a probe biomolecule on a substrate. The method comprises (i) introducing to the substrate a photoinducible charge separation moiety that effects charge separation upon photoinduction, an electron donor, and an electron acceptor, wherein the electron acceptor is capable of providing detectable photoreduction indicating the presence of the bound target molecule upon excitation of the charge separation moiety; (ii) photoinducing the charge separation moiety with energy sufficient to effect charge separation and provide detectable photoreduction of the electron acceptor; and (iii) measuring the detectable photoreduction. The steps can be performed sequentially, concurrently or in an overlapping fashion.

In preferred embodiments, charge separation is localized at the bound target and probe, so that detectable photoreduction is specific for the bound target and probe, and therefore distinguishable from photoreduction that might otherwise result due to the presence of unbound targets or probes. In one preferred embodiment, the charge separation moiety is localized at the bound target. In another preferred embodiment, a photosensitizer that promotes charge separation is localized at the bound target. Upon illumination of the photoinducible charge separation moiety in the presence of an electron donor and an electron acceptor, photoreduction is specifically detected at the location of the bound target.

The invention further provides for identification of multiple targets through spatial- or spectral-multiplexing. The invention can be used with optical scanning and analysis equipment, including inexpensive flatbed scanners, and with other routine chemical and electronic detection methods. No fluorescence detection is required.

Sensors of the invention are used to detect target biomolecules bound to a probe on a substrate and comprise a photoinducible charge separation moiety capable of transforming electromagnetic radiation (light) into an electron-hole pair (i.e., charge separation), an electron donor, and an electron acceptor. The photoinduced charge carriers participate in detectable redox reactions.

### Target and Probe Biomolecules

Biological molecules of the invention can be of any type, including nucleic acids, proteins, glycoproteins, polypeptides, hormones, cytokines, and biological reactants and reaction products. These terms are meant to be interpreted broadly and generally encompass organic or other molecules that are produced by biological systems or other biological molecules, are capable of affecting or altering biological systems when placed in contact therewith, and/or are capable of modifying or binding to other biological molecules. Biological molecules further include synthetic equivalents and substitutes.

As non-limiting examples, biomolecules include oligosaccharides, polysaccharides, oligopeptides, polypeptides, proteins, oligonucleotides and polynucleotides (e.g., DNA, cDNA, dsDNA, ssDNA). Biomolecules also include organic and organometallic compounds and salts thereof, saccharides, amino acids, nucleotides, lipids, carbohydrates, drugs, toxins, venoms, steroids, lectins, vitamins, minerals, hormones, cytokines, cofactors, coenzymes and metabolites.

Target and probe molecules can be any sort of complementary biological molecules or equivalents, including hybridizable nucleic acids, nucleic acids and DNA binding proteins, antibodies and antigens, receptors and ligands, heteromeric protein subunits, interacting protein pairs, etc., including those that have been conventionally used in connection with assays for detecting biological molecules, their interactions and functions. Complementary molecules can be bound covalently or in non-covalent complexes, for example, antibody-antigen complexes.

As will be appreciated by those of skill in the art, either the probe or target molecule may be immobilized on the array substrate for binding of probe and target in the context of the invention. For example, a sample containing suspected DNA targets may be denatured and hybridized to known single stranded probes spatially located on the array substrate. Alternatively, for example, sandwich assays may be used in which target molecules are immobilized by capture reagents affixed on the substrate and bound to probes.

Probes and/or targets are provided on substrates in patterns and linkages known to those of skill in the art. At least one probe is present; multiple probes can be employed to permit detection of multiple corresponding target molecules.

### Charge Separation Moiety

The invention makes use of photoinducible charge separation and generation of electron-hole pairs. Photoinducible charge separation moieties, in the context of the invention, are elements that effect charge separation upon excitation with electromagnetic radiation. More particularly, upon illumination with electromagnetic radiation with an energy higher than bandgap, positive (holes) and negative (electrons) charge carriers are produced which can participate in detectable redox reactions.

Charge separation is induced by subjecting the photoinducible charge separation moiety to electromagentic radiation that is sufficiently energetic to induce formation of electron-hole pairs. The photoinduced electrons and holes can reduce or oxidize materials, respectively, in the surrounding environment. Useful charge separation moieties include nanoparticles comprising metal oxides such as TiO₂, SnO₂, and WO₃, or mixtures of metal oxides that generate reactive electron-hole pairs upon photoirradiation. In a preferred embodiment, nanoparticles comprise TiO₂. For example, photoirradiation of TiO₂ nanoparticles with electromagnetic energy greater than bandgap (3.2 eV) creates electron-hole pairs.

Methods of making metal oxide nanoparticles useful in the invention are known in the art. See, e.g., Lettman et al., 2001, *Angew. Chem. Int. Ed.* 40:3160-3164; Zang, et al., 2000, *Chem. Eur. J*. 6, 379; Weller, 1993, *Angew. Chem. Int. Ed. Engl.* 32, 41; Henglein, 1988, *Top. Curr. Chem*., 143, 113; Henglein, 1989, *Chem. Rev.* 89, 1861; Bahncmann, in Photochemical Conversion and Storage of Solar Energy (eds. Pelizetti and Schiavello, 1991), p.251. In addition, metal oxide and custom-made mixed-metal oxide nanopowders are commercially available.

In different embodiments, as will be apparent to those skilled in the art, nanoparticles may be suspended, bound to target, probe, etc. or dispersed on a substrate or formulated in a film overlay. The nanoparticles may be adsorbed or linked to targets, probes or dyes. Where direct adsorption or linkage to TiO₂ or other charge separation moiety is not sufficient, nanoparticles may be modified to further comprise substances that modify physical characteristics of the nanoparticles, such as, for example, isoelectric points, chemical resistance, and adsorption and linkage capacities for biomolecules and biomarkers. Examples of such substances include, but are not limited to Al₂O₃, SiO₂, Ta₂O₅, Nb₂O₅, and ZrO₂.

The size of the nanoparticles can be from about 5 nm to about 500 nm. For particles dispersed on substrates or in coatings, larger particles provide greater sensitivity in measurement, whereas smaller particles provide greater dynamic range. Where nanoparticles are linked to probes, targets, dyes, or DNA intercalating agents, smaller particle sizes are preferred. Nanoparticles linked to biomolecules will generally have sizes from about 5 nm to about 200 nm, preferably from about 10 nm to about 100 nm, and more preferably from about 20 nm to about 40 nm.

The charge separation moiety can be tailored to modify its photoinduction characteristics. For example, doped nanoparticles can be particularly useful for certain applications. Doping provides higher quantum efficiency since a broader spectral range is available for photoinduced charge separation. Moreover, photoinduction is possible with ordinary lamps whereas, for example, pure TiO₂ or SnO₂ require UV irradiation. There is less potential for damage to probe and target molecules and to bonds conjugating biological molecules to the photocatalytic particles where UV radiation can be avoided. For applications where several different molecules are to be detected, the use of several different doped and undoped particles, that are photoinducible at different wavelengths, allows for color multiplexing.

A variety of dopants can be used to modify the bandgap characteristics of metal oxide particles. Three metal oxides (TiO₂, SnO₂, WO₃), which are not otherwise active when illuminated with visible light, are photoactivatable with visible light when doped with certain metals. For TiO₂, useful dopants include, for example, platinum (Pt), iridium (Ir), chromium (Cr), cobalt (Co), rhodium (Rh), ruthenium (Ru), terbium (Tb), manganese (Mn), and praseodymium (Pr). Like TiO₂, the bandgap for SnO₂ corresponds to an ultraviolet wavelength. SnO₂ is photoactivatable by visible light upon incorporation of many of the same dopants that are useful with TiO₂. A variety of other metals that are also useful as dopants include bismuth (Bi), calcium (Ca), certain transition metals, and certain lanthanoids. WO₃ has a bandgap energy in the visible spectrum, but depends on doping with, for example, iridium or chromium for photocatalytic activity. (See, Lettman et al., 2001).

### Electron Acceptors

Electron acceptors are substances that are reduced in redox reactions by photoinduced electrons and provide detectable photoreduction, for example, by observable precipitation or color change or by electrochemical means. Electron acceptors are reduced by photoelectrons in stoichiometric amounts, and are present, along with electron donors, in relative quantities sufficient to ensure that photoreduction is detectable. The electron acceptor is spatially proximate to the site of charge separation, providing detectable photoreduction at the site of the bound probe and target.

In certain embodiments, electron acceptors are metal ions in solution that precipitate when reduced by photoelectrons. Metal precipitation occurs on a substrate in the region where charge separation and photoreduction occurs. Hence, in preferred embodiments, where charge separation is localized at bound target and probe, the detection is specific for the bound target and probe and discriminates from unbound targets or probes. Precipitation onto a substrate is easily observed by optical detection of reflectance or absorbance. For example, metal precipitated onto a nylon membrane can be recorded and quantitated on an inexpensive optical flat-bed scanner. In some embodiments, the reducible metal ion is Ag⁺. Other precipitable metal ions include Pt⁺⁴, Au⁺², Hg⁺², and Cu⁺².

Alternatively, photoreduction of an electron acceptor is accompanied by a color change. For example, reduction of Cr⁺⁴ to Cr⁺³ in solution is accompanied by a color change. In another example, under conditions of sufficiently high pH, methyl viologen dication (MV⁺²) is reduced to the blue methyl viologen radical cation (MV⁺). Color development of a solution that results from photoreduction can be easily measured by colorimetric techniques such as are commonly employed, for example, in quantitation of enzyme linked immunosorbent assays.

The electron acceptor can also be an electrode in an electrochemical sensing device. For example, a charge separation moiety can be located at a target biomolecule bound on the surface of an electrode. When placed as an anode in an electrochemical sensing device, the electrode is an electron acceptor that captures photoelectrons produced upon illumination of the charge separation moiety. The current through the electrochemical device provides a measure of the amount of the bound charge separation moiety. Electrochemical sensing devices will usually include mediators, as described below, that provide electrons and act as charge carriers.

Electrodes can be conductors or semiconductors and be incorporated in substrates or coatings on substrates. Suitable materials for electrodes include, but are not limited to ITO, gold, silver, silicon and the like. Biological probes or capture reagents can be attached directly to electrodes or via linkers or polymeric coatings. Methods for forming working electrodes are known in the art. Exemplary methods are provided by Thorpe, et al., U.S. Patent 5,968,745.

### Electron Donor

Electron donors are substances that are oxidized in redox reactions by photoinduced holes. By donating electrons (scavenging holes) electron donors promote the transfer of photoelectrons to electrons acceptors and detectable photoreduction. In certain embodiments of the invention, the biological probe and target molecules are the electron donors. Alternatively, or in addition, other oxidizable organic molecules that readily donate electrons may be provided in working solutions. Such organic molecules include citric acid, salicylic acid, oxalic acid, and ethylenediamine tetraacetic acid (EDTA).

As indicated above, electrochemical sensors generally further comprise mediators which function as secondary electron donors and charge carriers. Mediators donate electrons, regenerating substances (e.g., biomolecules) oxidized in photoinduced redox reactions and are themselves regenerated by electrons from the cathode. As charge carriers, mediators promote detectable current flow between cathode and anode, and thus, through the electrochemical sensor. Mediators for use in the invention include conventional charge carriers employed in electrochemical devices, such as, for example organic conducting salts and viologen dyes. In some embodiments of the invention, the mediator is hydroquinone.

### Localization of Charge Separation

In preferred embodiments, charge separation is localized at a bound target and probe, so that the detectable photoreduction is specific for bound target and probe, by localization of a photoinducible charge separation moiety and/or a sensitizer of photoinduction. Otherwise, as will be appreciated, if detection is not specific for bound target and probe, the presence of unbound target or probe may need to be distinguished to account for any detectable photoreduction due to unbound probes or targets.

### Localization of Charge Separation Moiety

One way to localize charge separation at a bound target and probe is to link a charge separation moiety to the target or probe. In one embodiment, a substrate is provided to which is affixed a probe molecule that is specific for a target biomolecule of interest. A test composition comprising biomolecules linked to photoinducible charge separation moieties is contacted with the immobilized probe molecule, whereupon a target molecule, if present, is specifically bound. The test solution is removed and the substrate and attached probe analyzed for detectable photoreduction, indicating the presence of bound target.

In another embodiment, biomolecules of a test composition are immobilized on a substrate and assayed with a photoinducible charge separation moiety-linked probe that is specific for the target molecule of interest. Photoreduction indicates the presence of target molecules in the test composition.

In a third embodiment, typically referred to as a sandwich assay, a capture reagent and a probe linked to a photoinducible charge separation moiety are employed, each of which binds specifically to a target molecule of interest. The target molecule comprises a primary portion complementary to the probe and a secondary portion complementary to the capture reagent. The capture reagent is affixed to a substrate, and contacted with a test composition whereupon the target molecule, if present in the test composition, is bound by the capture reagent. The labeled probe is contacted with the substrate where it binds if the target molecule is present. Detectable photoreduction indicates the presence of the target molecule in the test composition.

Sandwich assays provide greater flexibility in selecting hybridization conditions and ordering binding reactions. For example, the kinetics for detection of a rare target molecule can be improved by first binding a probe to the rare target molecule in solution followed by capture of the probe-target hybrid on a substrate where photoinducible charge separation is detected.

A charge separation moiety can also be localized at a bound target and probe by linking it to a moiety that binds selectively to bound target and probe. For example, a photoinducible charge separation moiety can be made selective for doublestranded DNA (dsDNA) by linkage to an agent that intercalates between successive base pairs of dsDNA. The charge-separation moiety, and thus detectable photoreduction, is localized to regions of dsDNA.

A sensor according to this example comprises a single-stranded DNA probe immobilized on a substrate. A test composition is contacted with the immobilized probe such that complementary target biomolecules of interest can hybridize. The DNA intercalating agent binds to regions of dsDNA that are formed by probe/target hybridization as well as to regions of dsDNA that might already be present in the bound target molecule, thereby selectively locating a charge separation moiety at the bound target and probe.

Preferred DNA intercalating agents do not bind to single stranded DNA and include propidium iodide, ethidium bromide, 7-aminoactinomycin D and others that are well known in the art.

### Localization of Photosensitizer

In the context of this invention, photosensitizers function, when in proximity to a charge separation moiety, to reduce the energy required for charge separation, and thus, the wavelength of the illumination required for photocatalysis. In certain embodiments, photosensitizers can be adsorbed directly to a charge separation moiety. It will be apparent that doping is a form of photosensitization and that charge separation moieties with adsorbed photosensitizers have similar applications as doped nanoparticles.

A photosensitizer can also be localized at a bound target and probe by the same approaches as are provided above for charge separation moieties. The photosensitizer can be linked to a target biomolecule or linked to a probe or linked to a moiety that binds selectively to bound target and probe such as a DNA intercalating agent. In a variation, the photosensitizer is a DNA intercalating agent.

Where a photosensitizer is localized at a bound target and probe, charge separation can be localized even if the charge separation moiety is dispersed. For example, nanoparticles comprising TiO₂ or other metal oxides may be dispersed on substrates or incorporated into films or overlays that are deposited on substrates. The system is illumination at a wavelength that photoinduces nanoparticles in proximity to the photosensitizer, but not nanoparticles that are distributed elsewhere. Accordingly, detectable photoreduction occurs only in the vicinity of the photosensitizer.

A variety of photosensitizers are known in the art, including ruthenium (Ru) complexes, osmium (Os) complexes and pure organic dyes. See, e.g., Smestad, G. et al., 1994, *Sol. Energy Mater. Sol. Cells*, 32, 259. Among efficient dyes are complexes containing bipyridine-type ligands coordinated to Ru. Examples include [Ru(bpy)₃]⁺², Ru(H₂-dcbpy)₂NCS₂, [Ru(H₃-tctpy)NCS₃]⁻, and c*is-*RuL₂(SCN)₂ where L is 2,2'-bipyridyl-4-4'-dicarboxylate.

### Non-Specific Charge Separation

The invention also includes embodiments wherein detectable photoreduction is not localized at the bound target and probe. For example, biomolecules capable of acting as electron donors (e.g., ssDNA) can be immobilized on a substrate on which photoinducible charge separation moieties have been dispersed, and used to bind target molecules in a test sample. However, to determine the amount of target molecule, it may be necessary to differentiate detectable photoreduction that results from bound probe and target from photoreduction resulting from unbound probe.

### Substrate

Depending on the application, substrates can be conducting or nonconducting. Typical substrates are glass, indium tin oxide (ITO) -coated glass, gold-coated glass and silicon and various polymers. The substrates can be rigid or, alternatively, sheets, films or membranes. Polymeric substrates, have desirable properties such as light weight, toughness, moldability and flexibility. Examples of useful polymers include polyethylene terephalate (PET), poly(ether-ether-ketone) (PEEK) and Kapton™.

Unlike inorganic glasses and crystalline materials, polymeric substrates can be easily and rapidly shaped by use of thermoforming, photocuring, laser ablation and plasma etching. Such materials and processes are well known to those of skill in the art. Furthermore, the surface physical properties (e.g., wetting), chemical functionality and topography (e.g., porosity, roughness) can be easily tailored to the desired characteristics.

These and a wide variety of other materials, which are not compatible with fluorescence detection due to light scattering, opacity and background fluorescence, advantageously can be used in the present invention. The instant substrates are compatible with detection of metal deposition or colorimetric changes by measurement of light reflectance or transmission. The substrates can also be coated, for example with gold, ITO or other conductive substance to provide for electronic or electrochemical sensing of photoelectrons.

Useful substrates further include fibrous, microporous, or adsorbing materials such as paper, and nylon and nitrocellulose membranes, and other composite materials, which can be further coated or surface modified.

### Multiplexing

The instant invention is compatible with spatial- and spectral- multiplexing of assays of biological molecules. Multiplexing refers to the manner in which large numbers of assays are simultaneously performed on one or more biolobical samples, for example, using microarrays. "Spatial multiplexing" refers to the manner in which assays are arranged on microarrays in order to measure, many characteristic of a single test sample, or a single characteristic or many test samples, or multiple characteristics of multiple test samples.

For example, in a microarray such as a genechip, thousands of different DNA fragments of known sequence are immobilized at unique positions on a defined surface. When a sample is introduced to the array and hybridization is found to occur at specific array coordinates, the presence of a nucleotide sequence in the sample complementary to the sequence of the immobilized fragment at that location can be deduced.

In a second example, 100 samples are each tested for 10 biological molecules using an microarray having 1000 "microlocations." Each microlocation represents a combination of a single specific probe and single test sample.

Each of these examples are illustrative of the manner in which spatial multiplexing is employed in biological assays, and it will be evident to those of skill in the art that each of the above embodiments of the invention is compatible with identification of a bound target through spatial multiplexing.

The instant invention further provides for spectral multiplexing of biological assays. Spectral multiplexing refers, for example, to the simultaneous or nearly simultaneous assay of multiple biological targets at a single physical location. Color multiplexing is provided in the instant invention through the use of probe-linked charge separation moieties and/or photosensitizers having different spectral photoactivation characteristics. For example, different probes can be used, attached to charge separation moieties having different bandgap energies and thus, photoinducible with illumination of differing wavelengths, to detect different target molecules in the same sample at a single physical location. The presence of a particular bound target molecule among a set of target molecules is indicated by charge-separation at excitation wavelengths corresponding to the probe specific for that target molecule. Methods of modifying nanoparticle characteristics include, as noted above, incorporation of dopants and adsorption of sensitizing dyes. In an exemplary embodiment, where there are two probes, one having a charge-separator that requires ultraviolet light for photoinduction and the second having a charge-separator that is photoinducible over a broader range of energies, including visible wavelengths, illumination with UV light will result in charge separation by both labels, whereas visible light will be sufficiently energetic to photoinduce only the charge-separator that is sensitive to visible light. Thus, by choosing a first wavelength at which one charge-separator is active and a second wavelength at which both charge-separators are active, the presence of two different target molecules can be determined by comparing detectable photoreduction resulting from illumination at each of the wavelengths.

### Linkers

In those embodiments where target or probe biomolecules are adsorbed or linked to nanoparticle charge separation moieties, the biomolecules can be adsorbed or linked directly to the nanoparticles, or through functionalization or intermediate linking agents, as understood by those skilled in the art. A linking agent or functional group in the context of the present invention is any substance capable of binding or attaching one or more probe or target biomolecule with one or more nanoparticle.

Direct and indirect adsorption of biomolecules to substrates is well known in the art. Similarly, in certain embodiments of the invention, biomolecules may be directly adsorbed to TiO₂ containing nanoparticles or substrates. In other embodiments, the nanoparticles or substrates may be treated to facilitate absorption of biomolecules. Such methods are well known in the art. For example, polylysine is commonly adsorbed to a solid support to facilitate adsorption of nucleic acids.

Linkers include, but are not limited to, chemical chains, chemical compounds, carbohydrate chains, peptides, haptens, antibodies and the like. Linkers may vary in length and composition and properties such as flexibility, stability and resistance to chemicals, temperature, etc.

Biomolecules can be linked to nanoparticles by several methods, for example, including, but not limited to avidin/biotin or free chemical groups (e.g., thiol, carboxyl, hydroxyl, amino, amine, sulpho, etc.) and chemical groups reactive with those free chemical groups. For example, oligonucleotides can be functionalized with alkanethiols at their 3'-termini (See, Whitesides, Proceedings of the Robert A. Welch Foundation 39^{th} Conference on Chenical Research Nanophase Chemistry, Houston, TX, pp. 109-121 (1995)) or terminated with a 5' or 3' thionucleoside. Other functional groups for attaching oligonucleotides include phosphorothioate groups and substituted alkylsiloxanes. Methods for attaching biomolecules to nanoparticles include, for example, carboxylic acids on aluminum (Allara, 1985, *Langmuir* 1, 45), carboxylic acids on silica (Allara, 1974, *J. Colloid Interface Sci*., 410), silanes on silica (Maoz, 1987, *Langmuir*, 3, 1045), and carboxylic acids, aldehydes, alcohols and methoxy groups on titanium dioxide and silica (Lec, 1988, *J. Phys. Chem.* 92, 2597).

More than one biomolecule can be linked to a nanoparticle. The number of biomolecules per nanoparticle is limited only by steric effects. It will be appreciated by those of skill in the art that the size of the nanoparticles allows multiple binding interactions between a biomolecule linked to a nanoparticle and a biomolecule immobilized on a substrate.

Certain methods have the advantage that linkage to nanoparticles can be made subsequent to probe/target binding, particularly where the linkage is by a stable noncovalent interaction. For example, biotinylated probe molecules can first be bound to targets immobilized on a substrate, followed by attachment of avidin-linked nanoparticles.

It will be further appreciated that certain linkers allow complexes of charge separation moieties to be assembled. For example, avidin or an equivalent (e.g., streptavidin, ExtrAvidin®) may be bound to biotinylated probe bound to a target on a substrate in proper proportion so that, on average, each avidin molecule is bound to one biotinylated probe molecule. (Avidin has multiple biotin binding sites.) Multiple biotinylated nanoparticles can then bound at the remaining biotin binding sites.

The above described avidin-biotin binding scheme has the additional feature that large amounts of electron donating biomolecules and photoinducible charge separating nanoparticles can be localized to a single bound target and probe. Consequently, small amounts of bound target and probe can be efficiently detected.

Various detection methods have been described. In preferred embodiments, arrays of detectors are employed. The arrays can be locations on a membrane which are measured optically or arrangements of electrodes on a common surface, each electronically identifiable.

In an embodiment of the invention, electronically addressable microarrays are employed for sequential or simultaneous sample analysis of multiple samples. The number of addressable microlocations can be upwards of several million.

Throughout this application, various publications, patents, and patent applications have been referred to. The teachings and disclosures of these publications, patents, and patent applications in their entireties are hereby incorporated by reference into this application to more fully describe the state of the art to which the present invention pertains.

It is to be understood and expected that variations in the principles of invention herein disclosed may be made by one skilled in the art and it is intended that such modifications are to be included within the scope of the present invention.

## Claims

1. A sensor for detecting a target biomolecule bound to a probe biomolecule on a substrate compzising:
a photoinducible charge-separation moiety that effects charge-separation upon photoinduction,
an electron donor, and
an electron acceptor;
wherein the electron acceptor is capable of providing detectable photoreduction indicating the presence of the bound target molecule upon excitation of the charge-separation moiety.

2. The sensor of Claim 1, wherein the charge separation is localized at the bound target and probe.

3. The sensor of Claim 2, wherein the charge-separation moiety is localized at the bound target and probe.

4. The sensor of Claim 2, which further comprises a photosensitizer localized at the bound target and probe.

5. The sensor of any of Claims 1 to 4, wherein the charge-separation moiety comprises a metal oxide nanoparticle.

6. A method of detecting a target biomolecule bound to a probe biomolecule on a substrate comprising the steps of:
(i) introducing to the substrate a photoinducible charge-separation moiety that effects charge-separation upon photoinduction, an electron donor, and an electron acceptor, wherein the electron acceptor is capable of providing detectable photoreduction, indicating the presence of the bound target molecule upon excitation of the charge-separation moiety;
(ii) photoinducing the charge-separation moiety with energy sufficient to effect charge-separation and provide detectable photoreduction of the electron acceptor; and
(iii) detecting the photoreduction.

7. The method of Claim 6, wherein the charge separation is localized at the bound target and probe.

8. The method of Claim 7, wherein the charge-separation moiety is localized at the bound target and probe.

9. The method of Claim 7, which further comprises a photosensitizer localized at the bound target and probe.

10. The method of any of Claims 6 to 9, wherein the charge-separation moiety comprises a metal oxide nanoparticle.
